# EUROPÄISCHE PATENTANMELDUNG

(11) **EP 3 795 136 A1**
(43) Veröffentlichungstag der Anmeldung: **24.03.2021**
(21) Anmeldenummer: 19198622.3
(22) Anmeldetag: 20.09.2019
(51) Int. Cl.: A61K 8/25, A61K 8/46, A61K 8/9789, A61Q 5/00

(54) **HAARPFLEGEZUSAMMENSETZUNG ZUR VERMEIDUNG VON KOPFSCHUPPEN**

(71) Anmelder: Dr. Kurt Wolff GmbH & Co. KG, 33611 Bielefeld (DE)
(72) Erfinder: Menzel, Birgit, 33611 Bielefeld (DE); Albrecht, Harald, 33611 Bielefeld (DE)
(74) Vertreter: Ter Meer Steinmeister & Partner

(57) **Zusammenfassung**

Die vorliegende Erfindung betrifft eine Haarpflegezusammensetzung umfassend einen Pfefferkörnerextrakt, einen Rindenextrakt der Inga-Alba-Mimose, Silikapartikel und ein Tensidsystem, die lediglich 0,1 bis 11,0 Gew.-% Alkylethersulfate enthält. Des Weiteren gibt die vorliegende Erfindung eine kosmetische Verwendung dieser Haarpflegezusammensetzung zur Bekämpfung von Kopfschuppen an. Zuletzt ist die Erfindung auch auf die Haarpflegezusammensetzung zur Verwendung bei der Behandlung von pathologischen Zuständen der Kopfhaut gerichtet.

## Beschreibung

Die vorliegende Erfindung betrifft eine Haarpflegezusammensetzung umfassend einen Pfefferkörnerextrakt, einen Rindenextrakt der Inga-Alba-Mimose, Silikapartikel und ein Tensidsystem, die lediglich 0,1 bis 11,0 Gew.-% Alkylethersulfate enthält. Des Weiteren gibt die vorliegende Erfindung eine kosmetische Verwendung dieser Haarpflegezusammensetzung zur Bekämpfung von Kopfschuppen an. Zuletzt ist die Erfindung auch auf die Haarpflegezusammensetzung zur Verwendung bei der Behandlung von pathologischen Zuständen der Kopfhaut gerichtet.

Die menschliche Haut unterliegt einer ständigen Erneuerung. Abgestorbene Hautzellen werden als Schuppen abgestoßen. Dieser Vorgang geschieht im Normalfall unbemerkt, da die Schuppen sehr klein sind und nicht wahrgenommen werden. Ist die Zellerneuerungsrate erhöht, kann es allerdings dazu kommen, dass Hornzellen nur unvollständig ausreifen und Zellverbindungselementen (z.B. Corneodesmosomen) nur zum Teil abgebaut werden, so dass sich größere Verbunde an Hornzellen ablösen. Ebenso ist es möglich, dass die Schuppen verkleben und dadurch sichtbar werden. Kopfschuppen sind somit in erster Linie ein ästhetisches bzw. kosmetisches Problem. Das Phänomen kann jedoch auch von Juckreiz und Entzündungen begleitet werden und eine medizinische Behandlung erfordern.

Abhängig von der Ausprägung und den Begleiterscheinungen unterscheidet man einfache Kopfschuppen (*Pityriasis simplex*), fettige Kopfschuppen (*Pityriasis steatoides*), Asbestflechte (*Pityriasis amiantacea*) und Schuppen in Folge seborrhoischer Dermatitis (SD). Einfache Kopfschuppen äußern sich in trockenen weißen Schuppen, die sich leicht von der Kopfhaut lösen und förmlich herabrieseln. Fettige Hautschuppen und Schuppen, die bei seborrhoischer Dermatitis auftreten, sind gelblich gefärbt und kleben an der Kopfhaut oder an den Haaren. Unter Asbestflechte wird eine großflächige, dicke Schuppenbildung verstanden, die oft mit Haarausfall einhergeht. Darüber hinaus treten Kopfschuppen als ein Symptom der Schuppenflechte (Psoriasis) auf. Für die Schuppenflechte sind gerötete Hautareale mit silbrig-weißen Schuppen charakteristisch.

Kopfschuppen sind auf das Zusammenspiel mehrerer Faktoren zurückzuführen, deren Wirkungen in vielerlei Hinsicht noch nicht hinreichend erforscht sind. Sehr wahrscheinlich ist aber, dass die Malassezia-Hefen einen wesentlichen Beitrag zur Entstehung der Schuppen leisten. Diese Hefen, die in Form der Hefenstämme *Malassezia furfur* und *Malassezia globosa* vorkommen, sind neben Staphylokokken, Propionibakterien und weiteren Keimen ein natürlicher Bestandteil der mikrobiellen Flora einer gesunden Kopfhaut. Bei von Kopfschuppen betroffener Kopfhaut wurden Malassezia-Hefen jedoch in erhöhter Anzahl beobachtet. Die Population mit Malassezia-Hefen machte bei gesunder Kopfhaut nur um die 45% der gesamten Kopfhautflora aus, bei von Kopfschuppen betroffener Kopfhaut hingegen bis zu 75% und bei SD bis zu 83% (vgl. McGinley KJ, Leyden JJ, Marples RR, Kligman AM; Quantitative microbiology of the scalp in non-dandruff, dandruff and seborrheic dermatitis; J Invest Dermatol, 1975, 64; S. 401-405).

Einigkeit darüber, wie Malassezia-Hefen zur Entstehung von Kopfschuppen beitragen, existiert in der Fachwelt bisher nicht. Eine Theorie macht den Metabolismus der Hefen dafür verantwortlich (vgl. Turner GA, Hoptroff M, Harding CR, Stratum corneum dysfunction in dandruff, International Journal of Cosmetic Science, 2012, 34, S. 298-306). Da die Malassezia-Hefen keine eigene Fettsäuresynthetase besitzen, verstoffwechseln sie die Lipide, die von den Talgdrüsen auf der Kopfhaut produziert werden. Neben Diglyceriden, welche die Hefen zum Wachstum benötigen, fallen dabei auch ungesättigte Fettsäuren an, die von den Hefen wieder ausgeschieden werden und sich auf der Kopfhaut ablagern. Die ungesättigten Fettsäuren können zu Entzündungsreaktionen des Kopfhautgewebes führen. Dabei werden erhebliche Mengen an proinflammatorischen Cytokinen freigesetzt (vgl. Schwartz JR et al., A Comprehensive Pathophysiology of Dandruff and Seborrheic Dermatitis - Towards a More Precise Definition of Scalp Health, Acta Derm Venereol, 2013; 93, S. 131-137). Diese Faktoren begünstigen ihrerseits eine pathologisch erhöhte Proliferation des Kopfhautgewebes. Im schlimmsten Fall kommt es durch die Hyperproliferation sogar zu einer gestörten Barrierefunktion der Kopfhaut.

Vor diesem Hintergrund gibt es verschiedene Ansätze zur Bekämpfung von Kopfschuppen, die entweder separat oder kombiniert angewandt werden können.

Eine Herangehensweise setzt bei den Hornzellen an und bezweckt, die Zellansammlungen von der Kopfhaut abzulösen und aufzuspalten. Hierfür eignen sich Substanzen wie beispielsweise Salicylsäure, die ab einer Konzentration von 1% keratolytisch wirken (vgl. Trüeb RM, Shampoos: Ingredients, efficacy and adverse effects, JDDG, Band 5, 2007, S. 356-365; Melhorn S, Salicylsäure-Öle zur Anwendung auf der Kopfhaut, Der Hautarzt, 2017, 68, S. 248-249). Obwohl die Salicylsäure die Zugänglichkeit der Kopfhaut erleichtert und eine Behandlung mit weiteren Stoffen ermöglicht, ist sie mit Bedacht anzuwenden, da sie über die Haut resorbiert wird. Zudem ist bekannt, dass Salicylsäure leicht oxidativ abgebaut wird, wenn sie UV-Strahlung ausgesetzt wird (vgl. US 4 822 604 A). Zur Stabilisierung einer auf Salicylsäure basierenden Zusammensetzung sind also weitere Stoffe, wie zum Beispiel tertiäre Amine, nötig.

Eine andere Methode zur Bekämpfung von Kopfschuppen zielt darauf ab, das mikrobielle Gleichgewicht der Kopfhautflora wiederherzustellen und die Malassezia-Population auf ein normales Maß zu regulieren.

Der Artikel von Trüeb (vgl. Trüeb RM supra) berichtet in diesem Zusammenhang von Shampoos mit antimikrobiellen Mitteln wie Selendisulfid, Zink-Pyrithion, Pirocton-Olamin, Ketoconazol und Ciclopirox Olamin. Bei diesen Shampoos muss jedoch penibel auf die Häufigkeit der Anwendung und die Dosierung geachtet werden, um zu vermeiden, dass die gesamte Malassezia-Population abgetötet wird. Zudem inaktivieren die genannten Mittel nicht jeden Malassezia-Stamm. Gegen einige antimikrobielle Mittel haben die Hefen mittlerweile Resistenzen entwickelt, so dass eine entsprechende Behandlung ohne Wirkung bleibt. Hinzu kommt, dass die genannten antimikrobiellen Mittel nicht natürlichen Ursprungs sind. Das bedeutet, dass sie durch aufwendige mehrstufige Synthesen hergestellt werden müssen und gleichzeitig ein hohes Potential haben, weitere Hautreizungen zu verursachen. Letzterem kann durch die Zugabe von kühlenden Mitteln, wie sie in der US 2003/0161802 A1 vorgeschlagen wird, nur begrenzt begegnet werden. Zum einen kaschiert die Wirkung der kühlenden Mittel nur den Juckreiz und die weiteren Symptome der gereizten Haut. Zum anderen verschaffen die kühlenden Mittel dem Betroffenen nur kurzzeitig Linderung und die Entzündungssymptome werden nach Abklingen der kühlenden Wirkung noch stärker empfunden.

Des Weiteren offenbart Trüeb, dass Steinkohlenteer bei der Bekämpfung von Kopfschuppen eingesetzt werden kann. Obwohl der Wirkmechanismus nicht vollständig geklärt ist, hat sich gezeigt, dass Steinkohlenteer antientzündliche Eigenschaften hat. Steinkohlenteer ist eine zähflüssige dunkle Masse, die bei der Verarbeitung von Steinkohle gewonnen wird. Sie enthält verschiedene polyzyklische aromatische Kohlenwasserstoffe. Einige dieser polyzyklischen aromatischen Kohlenwasserstoffe stehen unter Verdacht, krebserregend zu sein. Wegen dem hohen Gesundheitsrisiko wird die Behandlung mit Steinkohlenteer heute nur noch selten angewendet. Zudem ist Steinkohlenteer als Bestandteil kosmetischer Shampoos seit geraumer Zeit in Deutschland verboten.

Die WO 2014/037529 A2 beschreibt eine Anti-Schuppen-Zusammensetzung, welche unter anderem 1-Acetoxychavicolacetat enthält. Dieser Wirkstoff kann aus *Alpinia galanga,* einem Thai-Ingwer, isoliert werden. Nachteilig an dieser Zusammensetzung ist aber, dass 1-Acetoxychavicolacetat nur anti-inflammatorische Eigenschaften hat. Indem es NF-κB hemmt, greift es in die Cytokin-Produktion der Zellen ein und verringert die Entzündungsreaktion des Körpers. Um die Malassezia-Population zu reduzieren, muss der Zusammensetzung aber mindestens noch ein weiterer antimikrobiell wirkender, nicht-natürlicher Stoff zugefügt werden.

Die US 4 931 274 A schlägt die Verwendung einer Zusammensetzung mit Tonerde zur topischen Anwendung auf einer von Schuppen betroffenen Kopfhaut vor. Die Tonerde enthält Magnesiumionen, die in wässriger Lösung desorbieren können und dann von der negativ geladenen Epidermis angezogen werden. Ein Teil der Magnesiumionen kann in die Epidermis eindringen und dort angeblich positiv auf die Zellerneuerungsrate einwirken.

Bei jedem der vorbeschriebenen Ansätze zur Bekämpfung von Kopfschuppen ist es wichtig, die Kopfhaut regelmäßig von Talg (Sebum) zu befreien. Dies sollte aber so schonend geschehen, dass eine zu massive Austrocknung der Kopfhaut vermieden wird. Amorphes, kolloides Silikondioxid ist bisher zur Behandlung von stark fettender Haut und Akne bekannt. Die US 4 536 399 A beschreibt, dass amorphes, kolloides Silika ("fumed silica") ein höheres Absorptionsvermögen für Öl besitzt als Kaolin, Aluminiumhydroxid und gefälltes Silica. Ein Artikel von Wilhelm et al. (Wilhelm KP, Cua AB, Wolff HH, Maibach HI, Surfactant-Induced Stratum Corneum Hydration in vivo: Prediction of the Irritation Potential of Anionic Surfactants, The Jounral of Investigative Dermatology, 101, 3, 1993, S. 310-315) geht auf die Wirkung ein, welche anionische Tenside auf die Haut haben. Gemäß diesen vorgestellten Untersuchungen führen N-Alkyl Sulfate initial zu einer hohen Wasseraufnahme der oberen Hautschicht. In kurzer Zeit gibt die obere Hautschicht das zusätzliche Wasser aber wieder ab, so dass ein Trockenheitsgefühl zurückbleiben kann.

Ausgehend hiervon war es Aufgabe der vorliegenden Erfindung, eine milde Haarpflegezusammensetzung bereitzustellen, welche antimikrobiell wirkt, Kopfschuppen deutlich reduziert und die Nachteile der bekannten Zusammensetzungen überwindet.

Diese Aufgabe wurde überraschend durch die Haarpflegezusammensetzung gemäß Anspruch 1 gelöst. Bevorzugte Ausführungsformen gehen aus den abhängigen Ansprüchen hervor.

Erfindungsgemäß wird die Aufgabe durch die Bereitstellung einer Haarpflegezusammensetzung gelöst, welche einen Pfefferkörnerextrakt, einen Rindenextrakt der Inga-Alba-Mimose, Silikapartikel und ein Tensidsystem umfasst, wobei die Zusammensetzung 0,1 bis 11,0 Gew.-% Alkylethersulfate enthält.

Der Pfefferkörnerextrakt ist auch als Piper Nigrum Berries Extract oder Piper Nigrum Fruit Extrakt bekannt. Piper nigrum ist eine Pflanzenart aus der Familie der Pfeffergewächse (Piperaceae), deren Früchte wegen des darin enthaltenen Alkaloids Piperin als scharf schmeckendes Gewürz verwendet werden. Zur Unterscheidung von ähnlichen Gewürzen spricht man auch vom echten Pfeffer. Anders als der Name vermuten lässt, sind die Früchte der Piper nigrum nicht immer schwarz. Je nach Erntezeitpunkt und weiterer Behandlung können die Pfefferkörner verschiedene Färbungen aufweisen (z.B. grün, schwarzer, weiß, rot).

Der Rindenextrakt ist ein Extrakt aus der Rinde eines Mimosengewächses. Die Mimosengewächse (Mimosoideae) sind eine Unterfamilie innerhalb der Familie der Hülsenfrüchtler (Fabaceae). Die 79 bis 82 Gattungen mit etwa 3275 Pflanzenarten sind fast weltweit, nämlich in tropischen bis subtropischen oder warm-gemäßigten Gebieten verbreitet. Inga alba ist eine Baumart aus der Unterfamilie der Mimosengewächse (Mimosoideae). Sie ist in Mittel- und Südamerika beheimatet.

Die Kombination der Extrakte kann kommerziell erworben werden, zum Beispiel von der BASF SE unter dem Namen Sanicapyl®.

Die Silikapartikel sind synthetisch hergestellte, amorphe Silikate. Sie fallen als Festkörper an und sind in Wasser unlöslich. Die Silikapartikel im Sinne der vorliegenden Erfindung sind besonders bevorzugt gefällte Silikapartikel. Sie werden aus Natriumsilikat (Wasserglas) durch Neutralisation mit Schwefelsäure erhalten. Die gefällten Silikapartikel werden auch "Hydrated Silica" (INCI-Bezeichnung) genannt und sind von den pyrogenen Silikapartikeln, die durch eine Flammenhydrolyse bei über 1000°C aus Siliziumtetrachlorid hergestellt werden, abzugrenzen. Die Silikapartikel werden unter verschiedenen Markennamen verkauft, z.B. Sipernat® und Spherilex® der Evonik Degussa GmbH.

Das Tensidsystem umfasst verschiedene Tenside. Unter diesen befinden sich in jedem Fall Alkylethersulfate, da diese anspruchsgemäß zu mindestens 0,1 Gew.-% in der Zusammensetzung enthalten sind. Maximal beträgt der Anteil der Alkylethersulfate an der Zusammensetzung 11,0 Gew.-%.

Diese Gewichtsanteile sowie alle im folgenden genannten Gewichtsanteile beziehen sich auf das Gewicht der gesamten Zusammensetzung, es sei denn es wird ausdrücklich eine andere Bezugsbasis angegeben.

Die Haarpflegezusammensetzung weist aufgrund der Kombination aus Extrakten, Silikapartikel und dem spezifischen Anteil an Alkylethersulfaten ein sehr gutes Reinigungsvermögen auf, verhindert aber gleichzeitig ein Austrocknen der Kopfhaut. Darüber hinaus wird bei regelmäßiger Anwendung der Zusammensetzung eine deutliche Reduktion der Kopfschuppen beobachtet. Die Haarpflegezusammensetzung wirkt zudem nachhaltig feuchtigkeitsspendend auf die Haut. Außerdem wird die Sebumproduktion, die insbesondere bei fettigen Kopfschuppen und Schuppen in Folge von SD erhöht ist, durch die Zusammensetzung vermindert. Zuletzt zeigt die Haarpflegezusammensetzung hervorragende antimikrobielle und antientzündliche Eigenschaften.

Somit eignet sich die Haarpflegezusammensetzung zur mehrmaligen wöchentlichen oder sogar zur täglichen Anwendung.

Die Haarpflegezusammensetzung ist bevorzugt ein Kopfhaut- und Haarreinigungssystem, besonders bevorzugt ein Anti-Schuppen-Shampoo.

In einer bevorzugten Ausführungsform ist der Pfefferkörnerextrakt in einem Anteil von 0,001 bis 0,1 Gew.-% und der Rindenextrakt in einem Anteil von 0,00006 bis 0,006 Gew.-%, jeweils bezogen auf das Gewicht der gesamten Zusammensetzung enthalten. Das Verhältnis zwischen Pfefferkörnerextrakt und Rindenextrakt ist vorzugsweise in einem Bereich von 10 : 1 bis 20 : 1, besonders bevorzugt von 15 : 1 bis 18 : 1.

Geringere Anteile der Extrakte von 0,001 Gew.-% Pfefferkörnerextrakt und 0,00006 Gew.-% Rindenextrakt können bereits das Wachstum des Hefenstammes *Malassezia furfur* inhibieren. Damit auch die Population des Hefenstammes *Malassezia globosa* auf ein normales Maß reduziert werden kann, sind höhere Gewichtsanteile von 0,01 Gew.-% und 0,0006 Gew.-% erforderlich.

Vorteilhaft ist, wenn die in der Zusammensetzung enthaltenen Silikapartikel eine spezifische Oberfläche von 100 bis 800 m²/g, bevorzugt von 150 bis 750 m²/g, besonders bevorzugt von 200 bis 700 m²/g, insbesondere von 280 bis 550 m²/g aufweisen (gemessen nach der BET-Methode in der ISO-Norm 9277). Diese hohen spezifischen Oberflächen sind charakteristisch für Partikel mit hoher Porosität. Durch die hohe Porosität können die Silikapartikel als Schwamm wirken. Ölige Substanzen, wie z.B. Lipide oder Sebum, können in den Poren der Silikapartikel aufgenommen bzw. absorbiert werden. Durch das Auswaschen des Haarpflegemittels werden diese Substanzen abtransportiert.

Die mittels Laserstreuung gemessene Partikelgrößenverteilung der Silikapartikel befindet sich bevorzugt in einem Bereich von 1 µm bis 1000 µm. Die mittlere Partikelgröße d₅₀ der Silikapartikel beträgt bevorzugt 5 bis 100 µm, besonders bevorzugt 5 bis 50 µm (gemessen nach der ISO-Norm 13320).

Weiterhin ist bevorzugt, wenn die Silikapartikel ein hohes Absorptionsvermögen aufweisen. Das mittlere Ölbindevermögen (die DOA-Absorption) ist bevorzugt 200-400 ml Di-Octyladipat pro 100 g Silikapartikel (gemessen nach der ISO-Norm 19246).

Die Silikapartikel sind vorzugsweise in einem Anteil von 0,01 bis 5,0 Gew.-%, besonders bevorzugt in einem Anteil von 0,05 bis 2,0 Gew.-%, ganz besonders bevorzugt in einem Anteil von 0,08 bis 1,6 Gew.-%, in der Haarpflegezusammensetzung enthalten.

In einer weiteren Ausführungsform enthält die Haarpflegezusammensetzung 2,0 bis 11,0 Gew.-%, bevorzugt 2,5 bis 10,0 Gew.-%, Alkylethersulfate. Die Alkylethersulfate sind besonders bevorzugt eine Mischung aus Laurylethersulfat und Myristylethersulfat. Eine Mischung aus Laurylethersulfat und Myristylethersulfat in diesem Anteil gewährleistet, dass die Haarpflegezusammensetzung mild zur Kopfhaut ist. Gleichzeitig zeigen die Alkylethersulfate in diesem Anteil keinen negativen Einfluss auf das Schäumverhalten und sind ausreichend, um eine gute Reinigungsleistung der Haarpflegezusammensetzung zu garantieren.

Die Zusammensetzung enthält vorzugsweise eine Mischung aus Laurylethersulfat, Myristylethersulfat, Sulfosuccinaten und Aminosäuretensiden. Dabei stellen Laurylethersulfat und Myristylethersulfat bevorzugt die Haupttenside dar, während die Sulfosuccinate und Aminosäuretenside den Co-Tensiden zuzurechnen sind.

Insgesamt kann die Haarpflegezusammensetzung mindestens 13,0 Gew.-%, bevorzugt mindestens 14,0 Gew.-%, besonders bevorzugt 14,5 bis 18,0 Gew.-%, anionische und nicht-ionische Tenside umfassen.

Weiterhin ist es vorteilhaft, wenn die Haarpflegezusammensetzung 8,0 bis 13,0 Gew.-%, bevorzugt 8,5 bis 12,5 Gew.-%, einer Mischung aus Alkylethersulfaten, Sulfosuccinaten und Acylglutamaten enthält. Bevorzugt machen die Sulfosuccinaten und Acylglutamaten zusammmen 1,5 bis 4,0 Gew.-% des Haarpflegezusammensetzung aus.

In einer Ausführungsvariante enthält die Zusammensetzung 1,0 bis 6,0 Gew.-%, bevorzugt 3,0 bis 5,5 Gew.-%, Polyethylenglykol-basierte Tenside. Die Polyethylenglykol(PEG)-basierten Tenside sind dabei beispielsweise ausgewählt aus der Gruppe bestehend aus PEG-120 Methylglucose Dioleat, PEG-3 Distearat, Laureth-2 und PEG-40 Hydrogenated Castor Oil.

PEG-120 Methylglucose Dioleat ist der PEG-Ether eines Veresterungsprodukts aus Ölsäure und Methylglucose (mit einem Verhältnis von Ölsäure zu Methylglucose von 2:1). Im Durchschnitt weist der Ether 120 Wiederholungseinheiten der Formel -CH₂-CH₂-O- auf. Bei PEG-3 Distearat handelt es sich um einen Diester der Stearinsäure (Octadecansäure), der mit der Summenformel C₄₂H₈₂O₆ angegeben werden kann. Zwischen zwei Stearinsäureresten befinden sich im Mittel 3 Wiederholungseinheiten der Formel -CH₂-CH₂-O-. Laureth-2 ist das Produkt einer Kondensationsreaktion zwischen Laurylalkohol und Ethylenoxid. Ein Molekül Laurylalkohol hat hierbei durchschnittlich mit 2 Molekülen Ethylenoxid reagiert. PEG-40 Hydrogenated Castor Oil ist die Bezeichnung für hydriertes, ethoxyliertes Rizinus-Öl.

Abgesehen von den PEG-basierten Tensiden, den Alkylethersulfaten, den Sulfosuccinaten und den Acylglutamaten kann die erfindungsgemäße Haarpflegezusammensetzung 0,05 bis 2,0 Gew.-%, bevorzugt 0,2 bis 1,5 Gew.-% weitere Tenside enthalten. Die weiteren Tenside können ausgewählt sein aus der Gruppe bestehend aus Zuckertensiden, Ölsäureestern, Milchsäureestern sowie Mischungen hiervon. Besonders bevorzugt sind die weiteren Tenside ausgewählt aus der Gruppe bestehend aus Coco-Glucosid, Glyceryl Oleat, Lauroyl Laktylat, Caproyl Laktylat sowie Mischungen hiervon.

Von diesen sind Coco-Glucosid und Glyceryl Oleat jeweils bevorzugt in einem Anteil von 0,05-0,8 Gew.-% in der Haarpflegezusammensetzung enthalten.

Darüber hinaus ist die Haarpflegezusammensetzung bevorzugt wasserbasiert. Das bedeutet, dass sie vorzugsweise 70,0 bis 85,0 Gew.-% Wasser umfasst.

In einer weiteren Ausführungsform enthält die Haarpflegezusammensetzung mindestens ein Additiv. Das mindestens eine Additiv kann in einem Anteil von 1,0 bis 7,0 Gew.-% vorliegen. Das mindestens eine Additiv kann ferner ausgewählt sein aus der Gruppe bestehend aus Haarkonditionierungsmitteln, Rückfettern, Konservierungsmitteln, Stabilisatoren, Duftstoffen, Antioxidantien, Rheologiemodifikatoren, Verdickungsmitteln, Pflegemitteln, Farbstoffen, Perlglanzmitteln, Aufhellungsmitteln, Lösungsmitteln und Kombinationen hiervon.

Als Antioxidatien kommen beispielsweise Ascorbinsäure, Tocopherol und Butylhydroxytoluol in Frage. Unter Lösungsmitteln können Co-Lösungsmittel verstanden werden. Alkohole, wie Butylen Glykol, sind Beispiele für Co-Lösungsmittel. Die Konservierungsmittel können ausgewählt sein aus der Gruppe bestehend aus Benzoesäure, Benzoesäurederivaten, Sorbinsäure, Sorbinsäurederivaten, Salicylsäure, Salicylsäurederivaten und Kombinationen hiervon. Haarkonditionierungsmittel können in pflanzlichen Ölen, Wachsen, Lecithinderivaten, Lanolinderivaten, quaternären Ammoniumverbindungen und Silikonen bestehen. Stabilisatoren können Farbstoffe oder andere lichtempfindliche Komponenten gegenüber Strahlung schützen und sind bevorzugt UV-Absorber wie zum Beispiel Benzophenonderivate. Als Rheologiemodifikator und Verdickungsmittel kann die Zugabe von Kochsalz (Natriumchlorid) in Betracht gezogen werden. Die Zugabe von Duftstoffen kann für einen angenehmen Geruch der Haarpflegezusammensetzung sorgen. Farbstoffe werden optional dazu verwendet, der Zusammensetzung eine charakteristische Farbe zu verleihen, so dass diese leicht von anderen Produkten unterschieden werden kann. Perlglanzmittel sind meist kristallin vorliegende Komponenten, die das Licht reflektieren und eine Haarpflegezusammensetzung brillant und gehaltvoll aussehen lassen.

Vorzugsweise enthält die Haarpflegezusammensetzung hydrolysiertes Weizenprotein und/oder Panthenol und/oder Allantoin und/oder Koffein und/oder Natriumbenzoat und/oder Kaliumsorbat und/oder Tocopherol und/oder Kochsalz und/oder Zitronensäure und/oder einen Farbstoff.

Hydrolysiertes Weizenprotein, Panthenol und Allantoin wirken pflegend für Kopfhaut und Haare. Hydrolysiertes Weizenprotein hat hauptsächlich feuchtigkeitsspendende Eigenschaften. Panthenol ist ein Provitamin welches im Körper zu Pantothensäure (Vitamin B5) umgewandelt wird. Letztere ist Teil des Coenzyms A und damit für den Hautstoffwechsel wichtig. Bei Einwirkung von Panthenol wird die Hautelastizität verbessert. Daneben werden Juckreiz und Entzündungen gelindert und die Wundheilung gefördert. Allantoin unterstützt ebenfalls die Wundheilung.

In diesem Zusammenhang ist es von besonderem Vorteil, wenn die Zusammensetzung 0,1-1,0 Gew.-% einer Mischung aus Natriumbenzoat und Kaliumsorbat und/oder 0,1-1,5 Gew.-% Parfüm und/oder 0,1-1,0 Gew.-% Zitronensäure und/oder 0,5-2,0 Gew.-% Kochsalz enthält.

Natriumbenzoat und Kaliumsorbat tragen zur Konservierung der Haarpflegezusammensetzung bei. Natriumbenzoat setzt in leicht saurem Milieu Benzoesäure und Kaliumsorbat Sorbinsäure frei. Beiden Säuren wird eine antimikrobielle Wirkung zugeschrieben.

Zitronensäure trägt in den angegebenen Mengen dazu bei, der Haarpflegezusammensetzung einen leicht sauren pH-Wert, bevorzugt von 4 bis 6, zu verleihen.

Durch die Zugabe von Kochsalz kann die Fließfähigkeit der Haarpflegezusammensetzung in gewissen Grenzen beeinflusst und auf das nötige Maß eingestellt werden. Zudem verbessert Kochsalz die Schäumbarkeit der Haarpflegezusammensetzung und trägt zu einem stabileren Schaum bei.

Bevorzugt ist weiterhin, wenn die Haarpflegezusammensetzung 0,1 bis 2,0 Gew.-% Koffein enthält. Koffein stärkt die Haarwurzeln, die bei von Kopfschuppen betroffener Kopfhaut häufig geschwächt sind, und ist daher ein wertvolles Additiv. Der stärkende Effekt basiert hauptsächlich auf der Hemmung des Enzyms Phosphodiesterase, die zu einem Anstieg des Wachstumsbotenstoffes cAMP führt. Besonders bevorzugt weist die Haarpflegezusammensetzung eine Konzentration von 0,5-1,5 Gew.-% auf.

Die vorliegende Erfindung bezieht sich weiterhin auf eine kosmetische Verwendung der vorbeschriebenen Haarpflegezusammensetzung zur Bekämpfung von Kopfschuppen. Ferner betrifft die vorliegende Erfindung die vorbeschriebene Haarpflegezusammensetzung zur Verwendung bei der Behandlung von pathologischen Zuständen der Kopfhaut.

Die pathologischen Zustände der Kopfhaut können auf eine atopische Dermatitis, eine seborrhoische Dermatitis, eine Psoriasis und/oder ein Ungleichgewicht in der mikrobiellen Besiedelung der Kopfhaut zurückgehen. Sie äußern sich in geröteten Hautarealen und einem anhaltenden Juckreiz.

Anhand der nachfolgenden Formulierungen soll die Erfindung verdeutlicht werden, ohne sie jedoch auf die speziellen Beispiele einschränken zu wollen.

Aus den in Tabelle 1 aufgeführten Komponenten wurden sechs verschiedene Rezepturbeispiele (A-F) für die erfindungsgemäße Haarpflegezusammensetzung hergestellt.

Bei der Herstellung der Rezepturbeispiele wurde zunächst Wasser vorgelegt (Verfahrensschritt 1). Anschließend wurden die Komponenten schrittweise hinzugefügt. In den Verfahrensschritten (2)-(5) wurden die folgenden Komponenten zugegeben:
(2) Konservierungsmittel, Koffein, Farbstoff, ggf. weitere Additive
(3) Tenside
(4) Sanicapyl®, hydrolysiertes Weizenprotein, ggf. weitere Additive
(5) Parfüm und ggf. weitere Additive

Nach jedem der Verfahrensschritte (2)-(5) wurde die Mischung in fachüblicher Weise homogenisiert. In einem weiteren Verfahrensschritt (6) wurde der pH-Wert und die Viskosität der Mischung eingestellt. Zuletzt wurden die Silikapartikel zugegeben und die Zusammensetzung wurde gemischt, bis die Silikapartikel homogen verteilt vorlagen.

Die Menge der Komponenten wurde jeweils so gewählt, dass ihr Gewichtsanteil in der fertigen Zusammensetzung den in Tabelle 1 angegebenen Gewichtsanteilen entspricht. In der Anwendung zeigte sich bei allen Rezepturen eine gute Hautverträglichkeit sowie eine deutliche Reduktion der Schuppen. Besonders gute Ergebnisse wurden mit Rezeptur A erzielt.

**Tabelle 1: Zusammensetzungen der Rezepturbeispiele.**

| **Komponenten** | **Rezeptur A** | **Rezeptur B** | **Rezeptur C** | **Rezeptur D** | **Rezeptur E** | **Rezeptur F** |
|---|---|---|---|---|---|---|
| Wasser | ad 100 | ad 100 | ad 100 | ad 100 | ad 100 | ad 100 |
| Natrium Laurylethersulfat | 7 | 7 | 5 | 6 | 7 | 5 |
| Natrium Myristylethersulfat | 3 | 2,5 | 1,5 | 1 | 0,5 | 1,5 |
| Dinatrium Laurylethersulfosuccinat | 1 | 1,5 | 1 | 1,5 | 1 | 1,5 |
| Natrium Lauroylglutamat | 1,5 | 1 | - | - | 1 | 2 |
| Natium Cocoylglutamat | - | - | 1,5 | 1 | - | - |
| Laureth-2 | 2 | 1,5 | 2 | 2 | 2 | 2 |
| PEG-120 Methylglucose Dioleat | 0,6 | 0,5 | 0,5 | 0,6 | 0,6 | 0,6 |
| PEG-3 Distearat | 2,25 | 2 | 2,5 | 2,25 | 2 | 1,5 |
| Hydrated Silica | 0,1 | 0,1 | 0,5 | 0,3 | 0,8 | 1,5 |
| Piper Nigrum Fruit Extrakt | 0,02 | 0,02 | 0,04 | 0,04 | 0,02 | 0,02 |
| Rindenextrakt der Inga Alba Mimose | 0,0012 | 0,0012 | 0,0024 | 0,0024 | 0,0012 | 0,0012 |
| Natrium Lauroyl Laktylat | 0,25 | 0,25 | 0,5 | 0,5 | 0,25 | 0,25 |
| Natrium Caproyl Laktylat | 0,2 | 0,2 | 0,4 | 0,4 | 0,2 | 0,2 |
| Butylenglykol | 0,53 | 0,53 | 1,06 | 1,06 | 0,53 | 0,53 |
| PEG-40 Hydrogenated Castor Oil | 0,2 | 0,2 | 0,1 | 0,2 | 0,1 | 0,1 |
| Glyceryl Oleate | 0,2 | 0,2 | 0,2 | 0,2 | 0,2 | 0,2 |
| Coco-Glucosid | 0,2 | 0,2 | 0,2 | 0,2 | 0,2 | 0,2 |
| Hydrolysiertes Weizenprotein | 0,5 | - | - | 0,1 | 0,2 | - |
| Panthenol | - | 0,1 | 0,2 | - | - | 1,5 |
| Allantoin | 0,2 | - | 0,2 | 0,1 | - | 0,1 |
| Koffein | 1 | 0,5 | 0,1 | 1 | 1,2 | 0,2 |
| Natriumbenzoat | 0,3 | 0,3 | 0,3 | 0,3 | 0,3 | 0,3 |
| Kaliumsorbat | 0,2 | 0,2 | 0,2 | 0,2 | 0,2 | 0,2 |
| Tocopherol | 0,1 | 0,05 | - | 0,05 | - | - |
| Parfüm | 0,5 | 0,7 | 0,7 | 0,7 | 0,7 | 0,6 |
| Kochsalz | 1,25 | 1,3 | 1,25 | 1,3 | 1 | 1,4 |
| Farbstoff | 0,1 | 0,1 | - | - | 0,1 | - |
| Zitronensäure | 0,4 | 0,5 | 0,4 | 0,5 | 0,6 | 0,4 |

## Patentansprüche

1. Haarpflegezusammensetzung umfassend
- einen Pfefferkörnerextrakt,
- einen Rindenextrakt der Inga-Alba-Mimose,
- Silikapartikel, und
- ein Tensidsystem,
wobei die Zusammensetzung 0,1 bis 11,0 Gew.-% Alkylethersulfate enthält.

2. Haarpflegezusammensetzung gemäß Anspruch 1, **dadurch gekennzeichnet, dass** der Pfefferkörnerextrakt in einem Anteil von 0,001 bis 0,1 Gew.-% und der Rindenextrakt in einem Anteil von 0,00006 bis 0,006 Gew.-%, jeweils bezogen auf das Gewicht der gesamten Zusammensetzung enthalten ist, wobei der Pfefferkörnerextrakt und der Rindenextrakt bevorzugt in einem Verhältnis von 10 : 1 bis 20 : 1, besonders bevorzugt von 15 : 1 bis 18 : 1, vorliegen.

3. Haarpflegezusammensetzung gemäß einem der vorhergehenden Ansprüche, **dadurch gekennzeichnet, dass** die Silikapartikel eine nach der BET-Methode gemessene spezifische Oberfläche von 100 bis 800 m²/g und/oder eine mittlere Partikelgröße d₅₀ von 5 bis 100 µm, besonders bevorzugt von 5 bis 50µm, und/oder ein mittleres Ölbindevermögen von 200-400 ml Di-Octyladipat pro 100 g Silikapartikel aufweisen.

4. Haarpflegezusammensetzung gemäß einem der vorhergehenden Ansprüche, **dadurch gekennzeichnet, dass** die Zusammensetzung 2,0 bis 11,0 Gew.-%, bevorzugt 2,5 bis 10,0 Gew.-%, Alkylethersulfate enthält, wobei die Alkylethersulfate besonders bevorzugt Laurylethersulfat und Myristylethersulfat sind.

5. Haarpflegezusammensetzung gemäß einem der vorhergehenden Ansprüche, **dadurch gekennzeichnet, dass** die Zusammensetzung mindestens 13,0 Gew.-%, bevorzugt mindestens 14,0 Gew.-%, besonders bevorzugt 14,5 bis 18,0 Gew.-%, anionische und nicht-ionische Tenside umfasst.

6. Haarpflegezusammensetzung gemäß einem der vorhergehenden Ansprüche, **dadurch gekennzeichnet, dass** die Zusammensetzung 8,0 bis 13,0 Gew.-% einer Mischung aus Alkylethersulfaten, Sulfosuccinaten und Acylglutamaten enthält.

7. Haarpflegezusammensetzung gemäß einem der vorhergehenden Ansprüche, **dadurch gekennzeichnet, dass** die Zusammensetzung 1,0 bis 6,0 Gew.-%, bevorzugt 3,0 bis 5,5 Gew.-%, Polyethylenglykol-basierte Tenside enthält.

8. Haarpflegezusammensetzung gemäß einem der vorhergehenden Ansprüche, **dadurch gekennzeichnet, dass** die Zusammensetzung 70,0 bis 85,0 Gew.-% Wasser enthält.

9. Haarpflegezusammensetzung gemäß einem der vorhergehenden Ansprüche, **dadurch gekennzeichnet, dass** die Zusammensetzung mindestens ein Additiv enthält, bevorzugt in einem Anteil von 1,0 bis 7,0 Gew.-%, wobei das mindestens eine Additiv besonders bevorzugt ausgewählt ist aus der Gruppe bestehend aus Haarkonditionierungsmitteln, Rückfettern, Konservierungsmitteln, Stabilisatoren, Duftstoffen, Antioxidantien, Rheologiemodifikatoren, Verdickungsmitteln, Pflegemitteln, Farbstoffen, Perlglanzmitteln, Aufhellungsmitteln und Kombinationen hiervon.

10. Haarpflegezusammensetzung gemäß einem der vorhergehenden Ansprüche, **dadurch gekennzeichnet, dass** die Zusammensetzung hydrolysiertes Weizenprotein und/oder Panthenol und/oder Allantoin und/oder Koffein und/oder Natriumbenzoat und/oder Kaliumsorbat und/oder Tocopherol und/oder Kochsalz und/oder Zitronensäure und/oder einen Farbstoff enthält.

11. Haarpflegezusammensetzung gemäß einem der vorhergehenden Ansprüche, **dadurch gekennzeichnet, dass** die Zusammensetzung 0,1-1,0 Gew.-% einer Mischung aus Natriumbenzoat und Kaliumsorbat, 0,1-1,5 Gew.-% Parfüm, 0,1-1,0 Gew.-% Zitronensäure und 0,5-2,0 Gew.-% Kochsalz enthält.

12. Haarpflegezusammensetzung gemäß einem der vorhergehenden Ansprüche, **dadurch gekennzeichnet, dass** die Zusammensetzung 0,1-2,0 Gew.-%, bevorzugt 0,5-1,5 Gew.-%, Koffein enthält.

13. Kosmetische Verwendung der Haarpflegezusammensetzung gemäß einem der vorhergehenden Ansprüche zur Bekämpfung von Kopfschuppen.

14. Haarpflegezusammensetzung gemäß einem der Ansprüche 1-13 zur Verwendung bei der Behandlung von pathologischen Zuständen der Kopfhaut.

15. Haarpflegezusammensetzung gemäß Anspruch 14, wobei die pathologischen Zustände auf eine atopische Dermatitis, eine seborrhoische Dermatitis, eine Psoriasis und/oder ein Ungleichgewicht in der mikrobiellen Besiedelung der Kopfhaut zurückgehen.
